# DEMANDE DE BREVET EUROPEEN

(11) **EP 0 950 352 A1**
(43) Date de publication de la demande: **20.10.1999**
(21) Numéro de dépôt: 99400716.9
(22) Date de dépôt: 24.03.1999
(51) Int. Cl.: A01K 67/00, A01K 1/02

(54) **Procédé pour améliorer les conditions d'élevage chez le porc nouveau-né**

(30) Priorité: 16.04.1998 FR 9804755
(71) Demandeur: L'AIR LIQUIDE, SOCIETE ANONYME POUR L'ETUDE ET L'EXPLOITATION DES PROCEDES GEORGES CLAUDE, 75321 Paris Cédex 07 (FR)
(72) Inventeur: Girardon, Philippe, 78000 Versailles (FR); Herpin, Patrick, Inst. Nat. Rech. Agronomique, 35590 L'Hermitage (FR)
(74) Mandataire: Mellul, Sylvie Lisette

(57) **Abrégé**

L'invention concerne un procédé pour améliorer les conditions d'élevage du porc selon lequel :
- on place le porcelet après la naissance dans une enceinte fermée ou semi-fermée ;
- on injecte dans l'enceinte un mélange gazeux comportant de l'oxygène, la teneur en oxygène du mélange gazeux étant comprise entre 25 et 50 %.

## Description

La présente invention concerne les procédés permettant d'améliorer les conditions d'élevage du porcelet (porc nouveau-né). On sait que la productivité des élevages porcins en France et dans le monde reste limitée par une mortalité post-natale élevée, cela malgré des améliorations intervenues ces dernières années concernant les techniques d'élevage, notamment en termes de nutrition et de maîtrise de l'ambiance et des pathologies de l'espèce.

Ainsi, à titre illustratif, les chiffres de taux de perte pour 1996 en France atteignent environ 18 % de la totalité des nouveau-nés, correspondant environ à 5 % de porcelets mort-nés, tandis que 13 % des porcelets nés vivants meurent entre la naissance et le sevrage, dont la moitié au cours des 48 premières heures suivant la naissance.

Si un certain nombre de causes ont été avancées pour expliquer ce taux de mortalité relativement élevé, causes telles que l'écrasement du nouveau-né par la truie, la sensibilité au froid ou encore certaines pathologies, reste que le quart de cette mortalité post-natale peut semble-t'il être attribué à un phénomène d'hypoxie résultant de la mise-bas, et affectant la fonction de thermorégulation et la vitalité du nouveau-né (voir par exemple l'article de P. Herpin et al paru aux Journées sur la Recherche Porcine en France en 1997, page 29).

En effet, lors de la mise-bas chez la truie, qui peut durer plusieurs heures, les contractions utérines répétées peuvent réduire les échanges entre le foetus et le placenta, ce qui peut entraîner une chute du débit sanguin à travers le placenta, et parfois même un décollement prématuré du placenta ou une rupture du cordon ombilical. On sait que ce risque augmente avec la durée de la mise-bas, avec la taille de la portée, mais également avec l'ordre de naissance au sein de cette portée.

On sait d'autre part que les porcelets les plus légers sont davantage sujets à ce phénomène d'hypoxie. Il faut noter également que même si les porcelets sont vivants après la mise-bas, ils peuvent souffrir et garder des séquelles de cette hypoxie néo-natale, car une anoxie de quelques minutes suffit pour inhiber les centres respiratoires et causer des lésions irréversibles dans le cerveau du porcelet.

Le degré d'hypoxie du porcelet à la naissance peut être notamment mesuré par l'un ou plusieurs des facteurs sanguins suivants :
- la pression partielle d'oxygène ou de CO₂ ;
- le pH ;
- la teneur en lactate.

Une hypoxie se caractérise alors par un pH bas, une lactatémie élevée, et une pression partielle de CO2 élevée.

On peut également signaler qu'une des conséquences du phénomène d'hypoxie chez le porcelet, par le fait qu'il diminue la vitalité physique de l'animal (stabilité, tentatives pour se lever), est de retarder le premier contact du porcelet avec la mamelle et donc la première tétée qui est nécessaire, non seulement, en terme d'apport d'énergie pour la thermo-régulation, mais également pour l'acquisition d'immuno-globulines.

Le document FR-A-2 710 816 propose un procédé et une installation pour améliorer les conditions d'élevage d'animaux en enceinte fermée ou semi-fermée, tel qu'utilisant une injection d'oxygène à intervalles périodiques ou lors de moments sélectionnés tels que les repas, les périodes de saillie, ou encore les périodes précédant ou suivant les naissances, la teneur en oxygène dans l'air ambiant étant alors maintenue entre 20,5 et 21,5 %. L'objectif visé par ce document est de résoudre un problème de mortalité des individus, notamment en période chaude, et par exemple pour les nouveau-nés lors de naissances prématurées ou après terme.

L'approche suivie par ce document, est de maintenir le taux d'oxygène autour de la teneur atmosphérique est donc d'ajuster cette teneur autour de 21 %, l'oxygène dans l'atmosphère environnante ayant en effet tendance à fortement diminuer dans les élevages intensifs du fait de la respiration des animaux.

L'approche de ce document s'attache donc plutôt à un renouvellement de l'atmosphère environnante qui s'appauvrit au fil du temps.

On peut citer également le cas du document FR-A-2 645 403, qui propose un procédé d'élevage d'êtres vivants en milieu oxygéné en vue d'en augmenter la taille, l'objectif étant de permettre d'augmenter la taille des animaux "jusqu'à des dizaines de fois" selon les espèces et "leur faire prendre plus de viande".

Outre que le document s'appuie sur des raisonnements forts peu scientifiques (comme d'évoquer la taille des libellules et autres grillons à l'époque du jurassique), il reste muet sur les conditions d'oxygène à appliquer pour atteindre de tels objectifs, et a fortiori ignore totalement les problèmes de mortalité post-natale.

La présente invention vise à résoudre les problèmes précédemment évoqués.

Pour ce faire, le procédé pour améliorer les conditions d'élevage du porc selon l'invention, selon lequel :
- on place le porcelet après la naissance dans une enceinte fermée ou semi-fermée ;
- on injecte dans l'enceinte un mélange gazeux comportant de l'oxygène ;
se caractérise en ce que la teneur en oxygène du mélange gazeux est comprise entre 25 et 50 %.

Le porcelet est donc selon l'invention, soumis, dans l'enceinte, à une atmosphère fortement suroxygénée puisque comportant entre 25 et 50 % d'oxygène.

Les travaux menés à bien par la Demanderesse ont donc permis de démontrer qu'il est avantageux, pour améliorer le taux de productivité d'un élevage porcin, non pas de procéder à un simple renouvellement d'air de l'élevage comme couramment rapporté dans la littérature, mais de procéder à une suroxygénation significative des nouveaux-nés puisque atteignant des teneurs de plusieurs dizaines de % d'oxygène.

Il est à noter que de façon surprenante, malgré de telles teneurs très élevées, on ne constate pas de toxicité de l'oxygène (poumons, voies aériennes.. ).

Le procédé selon l'invention peut par ailleurs adopter l'une ou plusieurs des caractéristiques suivantes :
- la teneur en oxygène du mélange gazeux est comprise dans la gamme allant de 30 à 50 %, et encore plus préférentiellement dans la gamme allant de 35 à 45 % ;
- le porcelet est placé dans l'enceinte dans l'heure qui suit la naissance ;
- le porcelet est placé dans l'enceinte dans les 30 mn qui suivent la naissance, et encore plus préférentiellement dans les 20 mn qui suivent la naissance, voire même immédiatement après la naissance ;
- le porcelet est maintenu dans l'enceinte, sous l'atmosphère constitué du mélange gazeux, pendant une durée de 5 à 30 mn ;
- le porcelet est maintenu dans l'enceinte, sous l'atmosphère constitué du mélange gazeux, pendant une durée de 10 à 25 mn ;
- le mélange gazeux, préalablement à l'injection dans l'enceinte, a été humidifié ;
- on met en oeuvre, durant tout ou partie de la mise-bas proprement dite, ledit mélange gazeux comportant 25 à 50 % d'oxygène.

D'autres caractéristiques et avantages de l'invention ressortiront de la description suivante donnée à titre illustratif sans caractère limitatif, en regard des dessins annexés sur lesquels :
- la figure 1 présente l'évolution comparative de la pression partielle d'oxygène sanguine en fonction du temps après la naissance (en minutes) pour deux lots de porcelets, un lot oxygéné après la naissance selon l'invention et un second lot témoin non traité selon l'invention ;
- la figure 2 présente l'évolution comparative du taux de lactate sanguin en fonction du temps après la naissance (en minutes) pour deux lots de porcelets, un lot oxygéné après la naissance selon l'invention et un second lot témoin non traité selon l'invention ;
- la figure 3 illustre les résultats obtenus en terme de température interne du porcelet (à la naissance, après 30 mn, et après 24 h) pour deux lots de porcelets, un premier lot oxygéné après la naissance selon l'invention et un second lot témoin non traité selon l'invention ;
- la figure 4 illustre les résultats comparatifs de taux de mortalité après 24 h, 48 h, 7 jours et 21 jours, ici encore pour deux lots de porcelets, un premier lot oxygéné après la naissance selon l'invention et un second lot témoin non traité selon l'invention.

Dans tous les cas de porcelets traités selon l'invention, les animaux ont été introduit 4 minutes après la naissance, dans une enceinte comportant de l'azote et une teneur en oxygène de 40%, et sont demeurés dans cette enceinte durant environ 20 minutes.

Le traitement est appliqué « intra-portée », c'est à dire qu'au sein d'une même portée, le même nombre de porcelets est affecté à chaque lot et qu'après le traitement , les porcelets traités selon l'invention sont replacés avec leur mère et élevés dans les mêmes conditions que les porcelets témoins.

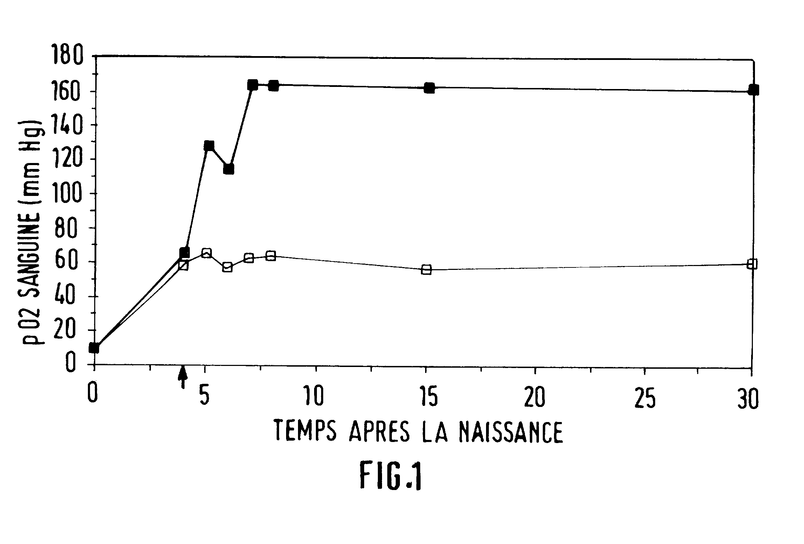

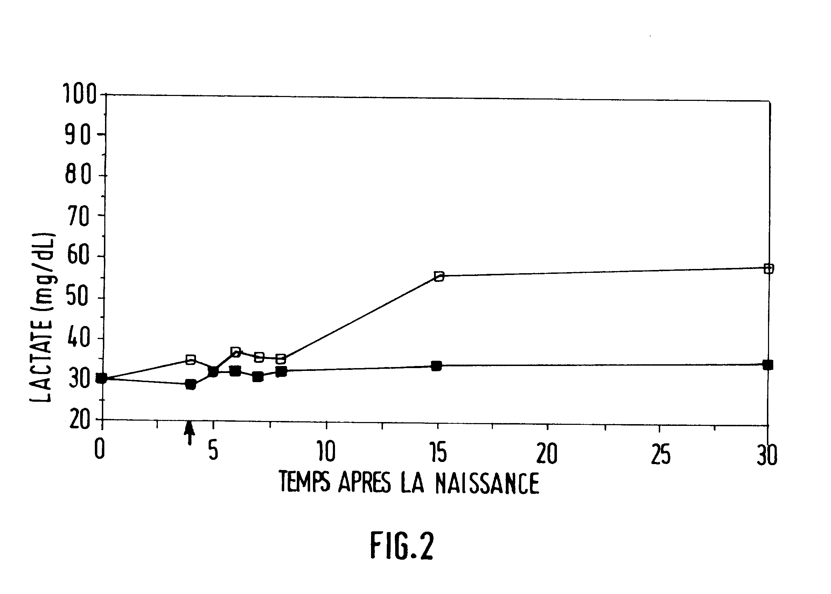

On voit donc au niveau des figures 1 et 2, une flèche sur l'axe des abscisses pointant 4 minutes, et représentant le moment suivant la naissance où le porcelet est entré dans l'enceinte pour être mis en contact avec l'atmosphère de traitement.

Pour ces deux figures, la courbe en carrés pleins a été obtenue pour le lot de porcelets oxygéné selon l'invention, tandis que la courbe en carrés vides a été obtenue pour le lot de porcelets témoin.

Les résultats illustrés pour ces deux figures, en terme de pression partielle d'oxygène sanguine et en terme de taux de lactate sanguin, démontrent clairement une amélioration de l'oxygénation des tissus (pression partielle d'oxygène augmentée par le traitement, augmentation post-natale du taux de lactate maîtrisée), et donc une influence sensible sur le métabolisme énergétique de l'animal.

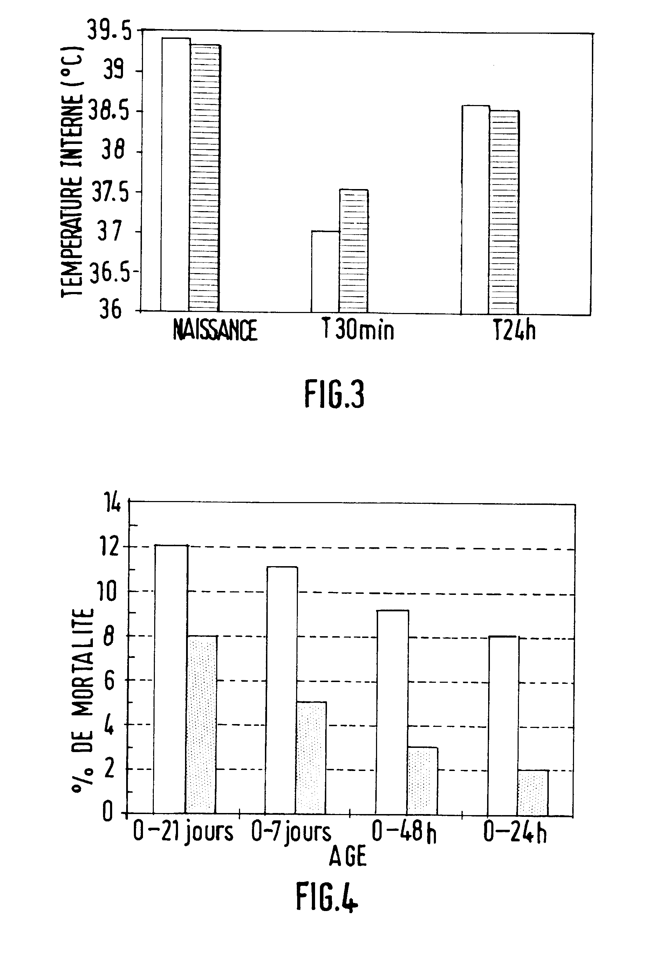

Venons en maintenant aux résultats de température interne représentés en figure 3, pour lesquels les résultats du lot témoin sont représentés en barres vides tandis que les résultats du lot selon l'invention sont représentés en barres pleines.

De façon tout à fait cohérente, ces résultats de température interne montrent que la température interne du porcelet chute naturellement au cours des 30 premières minutes suivant la naissance, avant de remonter progressivement grâce à la fonction de thermorégulation (voir évolution des barres vides).

On constate que le traitement d'oxygénation selon l'invention limite significativement la température mesurée après 30 mn, le traitement selon l'invention supprimant on l'a vu, les effets négatifs de l'asphyxie mais améliorant également alors les capacités thermo-régulatrices immédiatement après la naissance.

On peut penser que le traitement d'oxygéno-thérapie appliqué stimule la fonction de thermo-régulation favorisant le métabolisme oxydatif, c'est-à-dire l'oxydation du glucose en réduisant la production de lactate, ce qui permet une production plus importante d'énergie au moment où les dépenses énergétiques du porcelet sont maximales.

Après 24h, les résultats de température des deux lots sont comparables, mais on comprend que ces résultats ne concernent que les animaux qui ont survécu.

Or, comme illustré figure 4 (même représentation des deux lots que pour la figure 3), le taux de survie des porcelets après 24 h, 48 h, 7 jours et 21 jours (c'est-à-dire au sevrage) s'en trouve très nettement amélioré : le taux de mortalité étant réduit d'un tiers après 21 jours.

On notera d'ailleurs que le traitement d'oxygénation selon l'invention n'a pas d'effet sur le poids des individus ayant survécu après sevrage, on obtient donc pour les lots testés le même poids à 21 jours et la même croissance jusqu'au stade d'abattage pour le lot témoin et pour le lot oxygéné selon l'invention.

Quoique la présente invention ait été décrite en relation avec des modes de réalisation particuliers, elle ne s'en trouve pas limitée pour autant mais est au contraire susceptible de modifications et de variantes qui apparaîtront à l'homme de l'art dans le cadre des revendications ci-après.

## Revendications

1. Procédé pour améliorer les conditions d'élevage du porc, selon lequel :
- on place le porcelet après la naissance dans une enceinte fermée ou semi-fermée ;
- on injecte dans l'enceinte un mélange gazeux comportant de l'oxygène ;
caractérisé en ce que la teneur en oxygène du mélange gazeux est comprise entre 25 et 50 %.

2. Procédé selon la revendication 1, caractérisé en ce que la teneur en oxygène du mélange gazeux est comprise dans l'intervalle allant de 30 à 50 %.

3. Procédé selon la revendication 1 ou 2, caractérisé en ce que la teneur en oxygène du mélange gazeux est comprise dans l'intervalle allant de 35 à 45 %.

4. Procédé selon l'une des revendications 1 à 3, caractérisé en ce que le moment où l'on place le porcelet dans l'enceinte est situé dans l'heure qui suit la naissance.

5. Procédé selon la revendication 4, caractérisé en ce que le moment où l'on place le porcelet dans l'enceinte est situé dans les 30 mn qui suivent la naissance.

6. Procédé selon la revendication 4 ou 5, caractérisé en ce que le moment où l'on place le porcelet dans l'enceinte est situé dans les 20 mn qui suivent la naissance.

7. Procédé selon l'une des revendications 1 à 6, caractérisé en ce que le porcelet est maintenu dans l'enceinte sous atmosphère pendant une durée de 5 à 30 mn.

8. Procédé selon la revendication 7, caractérisé en ce que le porcelet est maintenu dans l'enceinte sous atmosphère pendant une durée de 10 à 25 mn.

9. Procédé selon l'une des revendications 1 à 8, caractérisé en ce que le mélange gazeux injecté dans l'enceinte a été au préalable humidifié.
